(19) Europäisches Patentamt

European Patent Office

Office européen des brevets

(11) **EP 1 234 541 B1**

(12) **EUROPEAN PATENT SPECIFICATION**

(45) Date of publication and mention
of the grant of the patent:
**26.05.2004 Bulletin 2004/22**

(51) Int Cl.$^7$: **A61B 5/083**

(21) Application number: **01830120.0**

(22) Date of filing: **22.02.2001**

(54) **Machine and method for measuring oxygen consumption**

Gerät und Verfahren zur Messung des Sauerstoffverbrauchs

Dispositif et procédé pour mésurer la consommation de l'oxygène

(84) Designated Contracting States:
**AT BE CH CY DE DK ES FI FR GB GR IE IT LI LU MC NL PT SE TR**

(43) Date of publication of application:
**28.08.2002 Bulletin 2002/35**

(73) Proprietor: **UNIVERSITA DEGLI STUDI DI BOLOGNA**
**40126 Bologna (IT)**

(72) Inventor: **Zannoli, Romano**
**San Salvatore di Casola, 40065 Pianoro (IT)**

(74) Representative: **Jorio, Paolo et al**
**STUDIO TORTA S.r.l.,**
**Via Viotti, 9**
**10121 Torino (IT)**

(56) References cited:
**DE-A- 2 035 982**       **US-A- 4 856 531**
**US-A- 5 285 794**

- **MALTZAHN VON W W ET AL: "OXYGEN CONSUMPTION MONITOR FOR INFANTS" PROCEEDINGS OF THE ANNUAL INTERNATIONAL CONFERENCE OF THE IEEE ENGINEERING IN MEDICINE AND BIOLOGY SOCIETY,US,NEW YORK, IEEE, vol. 16, 3 November 1994 (1994-11-03), pages 856-857, XP000552369 ISBN: 0-7803-2051-4**

## Description

The present invention relates to a machine for measuring the oxygen consumption of a living creature.

The present invention may be used to advantage in the field of human physiology, to which the following discussion will refer explicitly without for this reason losing its general character.

In the physiological field it is known that the function of the heart is to pump blood into the body circulation with a sufficient flow rate to keep the tissues in a healthy condition. In other words, the heart must produce a sufficient output of blood to cope with the body's nutritious needs, particularly of oxygen, and to remove the metabolism waste products from the tissues.

The cardiac output (C.O.), defined as "the blood volume ejected per minute by the left ventricle of the heart", is the combination of two factors. The first is the heart rate (H.R.), that is the number of beats per minute (bpm), the second is the unitary (systolic) volume or stroke volume (S.V.), that is the quantity of blood pumped by the ventricle for each stroke, measured in millimetres (A.M. Katz, Physiology of the heart, Raven Press, 1977).

The studies that have been carried out on certain mammals, particularly on man, have demonstrated that there is a linear relationship between cardiac output and oxygen consumption during breathing (A.C. Barger, V. Richards, J. Metcalfe and B. Günter, Regulation of circulation during exercise, Am. J. Physiol. 184: 613-623, 1956): the organism under stress needs a greater quantity of oxygen, which is supplied by a sudden adaptation of the cardiac output.

So, in healthy subjects it is possible to estimate their cardiac output by measuring their oxygen consumption; in subjects with heart disease or dysfunctions of the respiratory system, it is possible to observe how this relation diverges from simple linearity. In particular, estimates of oxygen consumption are useful for improving athletic performances, among other things allowing identification of the anaerobic threshold of the individual subjects. In fact, it is possible to identify the anaerobic threshold of a subject by observing the trend of oxygen consumption and of the production of carbon dioxide during a programmed progressive effort.

At present, to find the oxygen consumption, a device is used which measures both the ventilation and the concentration of oxygen in the air ventilated by the patient. The most sophisticated method performs a "breath by breath" analysis and comprises a rapid analyser for measuring the concentration of $O_2$ and of the flow of gases relating to each breathing action, directly at the subject's mouth. Multiplying instant by instant the air flow F(t) by the oxygen concentration C(t), the instantaneous oxygen flow is obtained which, integrated on the interval of a breath, provides the quantity of oxygen dissolved in the blood for each breath. Multiplying again by the breathing frequency, the volume of consumed oxygen $\dot{V}O_2$ per minute is obtained.

$$\dot{V}O_2 = \int_T F(t) \cdot C(t)dt$$

The error associated with the measurement depends on the precision of the flow meters and on the sensitivity and response speed of the oxygen analysers.

The flow is measured using spirometers, pneumotacographs, mass flow meters, convection flow meters, ultrasound flow meters, turbines. These devices do not always guarantee a correct instantaneous measurement (turbines especially present delayed responses). The instantaneous measurement of the concentration of oxygen in the ventilated air may be carried out with a mass spectrometer, with electrochemical or magnetic systems. In the specific case of breath by breath measurement it is indispensable that the response of the oxygen analyser be suitably fast and that there be no time difference between the concentration signal and the flow signal.

Only the mass spectrometer has the necessary response speed to detect with precision the continuous modifications of the concentration during a single breathing action. However, this type of rapid analyser has the inconvenience of requiring accurate calibration with a reference gas, high stabilisation times, high cost and it must be used by expert operators. Other analysers, which use electrochemical methods (zirconium cells) and paramagnetic methods, have a sufficiently rapid response and, with a little artifice, they may be used in breath by breath procedures.

Electrochemical analysers with a longer response time (even a few seconds) may be used only with collection of the gases in mixing chambers and, unless particular precautions are taken, they are not suitable for measurement during effort.

All the devices for analysing oxygen consumption during effort present the limit of requiring the use of a mask or a mouthpiece. The need to apply the mask or mouthpiece on the subject to measure the ventilation and collect the exhaled air is the greatest limitation on the use of oxygen consumption measurement. These accessories in fact imply complex and costly washing and sterilising procedures, or the use of disposable material that is not always adequate and has a high cost. Furthermore, some subjects do not support the mask or mouthpiece well, and consequently present irregular breathing which distorts the measurements.

For example, US 5285794 discloses a method of monitoring the respiratory gas of a patient including a gas mixing chamber. A constant flow of a therapeutic gas mixture is measured by a flow meter in a supply line leading to a face mask breathing device. The mask is by-pass connected to the supply line such that the patient inspires from and exhales into the flow from the supply line. Both by-pass and expired gas mix and enter the adjustable-volume chamber, which contains an internal fan and sensors for detecting percentage content of oxygen and carbon dioxide.

As another example, DE 2035982 discloses an apparatus for monitoring the respiratory gas of a patient including a gas mixing chamber and a face mask breathing device.

The object of the present invention is to produce a machine for measuring oxygen consumption which is free from the inconveniences described above and is at the same time easy and economic to use.

According to the present invention a machine for measuring oxygen consumption is realised with the features as claimed in claim 1.

The present invention further relates to a method for measuring oxygen consumption

According to the present invention a method for measuring oxygen consumption is supplied with the features as claimed in claim 15.

The present invention will now be described with reference to the enclosed drawings which illustrate a preferred but not limiting embodiment, and in which:

-   figure 1 illustrates schematically and in perspective a preferred embodiment of a machine realised according to the present invention;

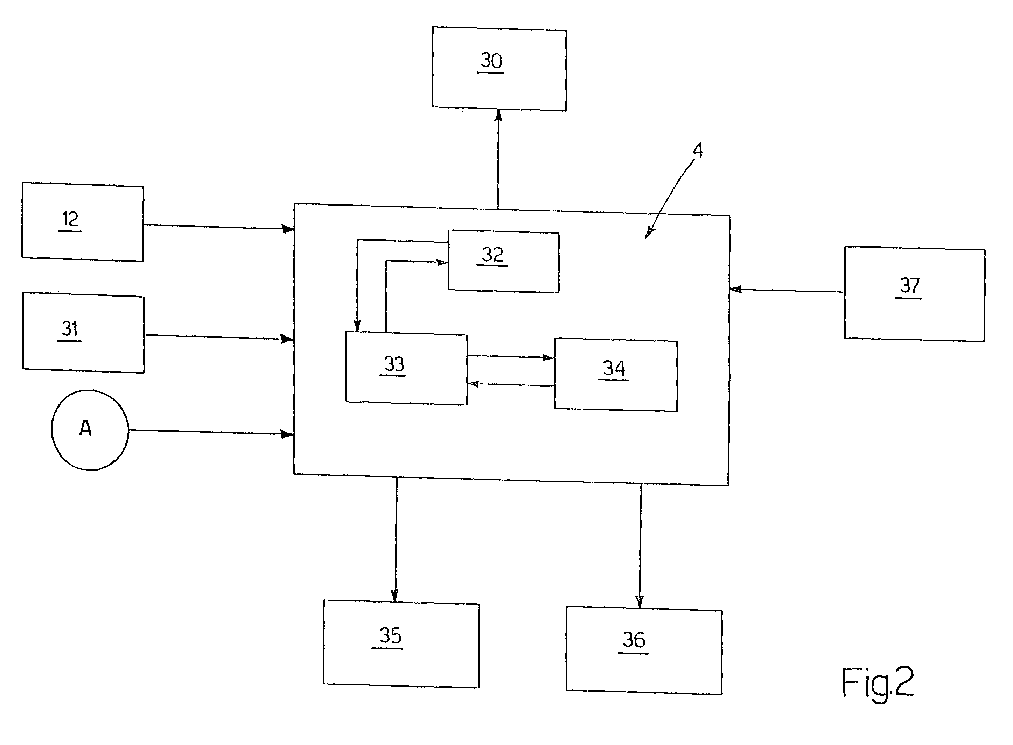

-   figure 2 illustrates schematically and in blocks some elements of the machine realised according to the present invention;
-   figure 3 illustrates schematically and in perspective a variation of a detail of the machine realised according to the present invention;
-   figure 4 illustrates schematically and in layout an alternative embodiment realised according to the present invention.

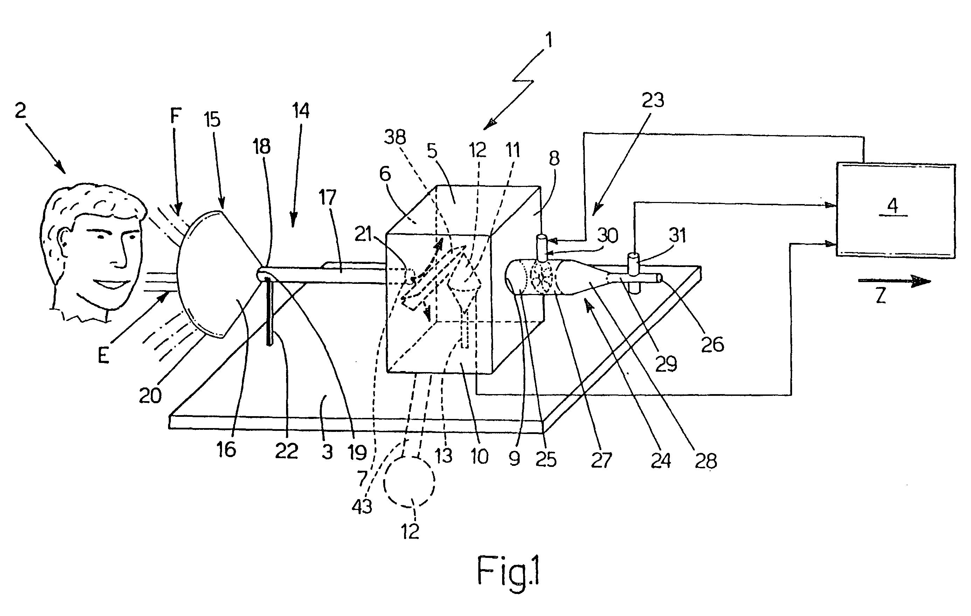

With reference to figure 1, indicated overall with 1 is a machine for measuring oxygen consumption by a subject 2. The concept of the machine 1 starts off from the theoretical and experimental observation of flow profiles near the mouth during forced breathing. It has in fact been found that, during inhaling, the air is taken from a wide peri-oral surface while during exhaling the air is concentrated in a jet with a limited volume, directed forwards.

This difference in behaviour means that, with the next intake of breath, only a very small part of the exhaled air is re-inhaled.

The machine 1 is positioned on a surface 3 and comprises a control device 4, of a prior art and schematically illustrated, and a mixing chamber 5 which is substantially box shaped. The mixing chamber 5 comprises a first vertical wall 6, with an entrance hole 7, and a second vertical wall 8 substantially parallel to the wall 6 and with an outlet hole 9. The holes 7 and 9 are coaxial and are defined in the centre of the respective walls 6 and 8. The line joining the centres of the holes 9 and 7 defines a horizontal axis Z substantially parallel to the surface 3.

The mixing chamber 5 further comprises a lower horizontal wall 10, substantially parallel to the surface 3 and lying on the surface 3 itself.

Inside the mixing chamber 5, more precisely in its centre 11, is installed a concentration detector 12 of a prior art and schematically illustrated. The detector 12 is supported by a bracket 13 integral with the horizontal wall 10 of the mixing chamber 5. The detector 12 is electrically connected to the control device 4.

According to a preferred embodiment the concentration detector 12 measures the oxygen concentration and, in particular, it is a paramagnetic oximeter. This instrument exploits the behaviour of oxygen exposed to an intense magnetic field: in these conditions the oxygen tends to remain in the zone of the magnetic field itself, determining in the pipe a resistance to the flow which contains it (R. Zannoli, C. Orsi, Elementi di strumentazione medica, Società Editrice Esculapio, 1995). This resistance is shown with a difference of pressure detected by a transducer and transformed into an electric signal sent to the control device 4.

According to a further embodiment the concentration detector 12 measures, in a known way, the concentration of carbon dioxide with an infrared technique.

The machine 1 further comprises an entrance conveyor 14 suited for conveying the gaseous substances exhaled by the subject 2 and the air coming from the surrounding environment into the mixing chamber 5. In figure 1 the jet of exhaled air is schematically represented by the hatching indicated with letter E, while the air coming from the outside environment is schematically represented by the alternate hatching indicated with letter F.

The above-mentioned conveyor 14 is equipped with an element 15 in the shape of a truncated cone, hollow on the inside and having the walls 16 preferably made of nylon. Said walls 16 are structured so as to be able to be partly deformed, thus causing variation of the volume of the element 15 itself, under the pressure of the air exhaled by the subject 2.

The conveyor 14 further comprises a flexible pipe 17, which extends substantially in the direction of the axis Z. The pipe 17 is situated upstream from the mixing chamber 5 and downstream from the element 15 so as to join the element 15 to the mixing chamber 5. The fist end 18 of the pipe 17 is joined to one of the bases 19 and 20 of the element 15, specifically the one with the shorter radius 19, the second end 21 is inserted through the entrance hole 7 into the mixing chamber 5.

A support 22, of a prior art and schematically illustrated, is positioned at the connection between the pipe 17 and the element 15. The support 22 is suited for maintaining the pipe 17 in a substantially horizontal position with respect to the supporting surface 3 and the conveyor 14 in a suitably position with respect to the subject 2.

The machine 1 further comprises an outlet conveyor 23 positioned downstream from the mixing chamber 5 and suited for conveying the gaseous substances present inside the chamber 5 towards the outside environment. The conveyor 23 is equipped with a pipe 24 with variable cross-section which extends substantially in the direction of the axis Y. The first end 25 of the pipe 24 is connected to the hole 9 and the second end 26 of the pipe 24 is free.

The pipe 24 comprises three sections 27, 28 and 29. The first section comprises the end 25, is directly connected to the mixing chamber 5 and has a substantially constant cross-section. The second section 28, located between the first section 27 and the third section 29, has a cross-section which decreases passing from the first section 27 to the third section 29. The third section 29 comprises the end 26 and has an internal diameter substantially constant smaller than the internal diameter of the first section 27 and substantially the same as the smallest internal diameter of the second section 28.

The outlet conveyor 23 further comprises a suction unit 30, of a prior art and schematically illustrated, situated at the section 27 and commanded by the control device 4. This suction unit 30 is suited for moving the gaseous substances, taking them from the conveyor 14, carrying them along the pipe, through the mixing chamber 5 and the pipe 24 and finally releasing them in the outside environment.

According to a preferred embodiment, the suction unit 30 comprises a fan driven by an electric motor.

The outlet conveyor 23 is also equipped with a flow meter 31, of a prior art and schematically illustrated, positioned downstream from the suction unit 30 at the third section 29; the flow meter 31 being connected to the control device 4.

According to a preferred embodiment the flow meter 31 is a Fleisch pneumotacograph.

The control device 4, electrically fed by means of a power supply line A, receives the measurements made by the concentration detector 12 and by the flow meter 31 and, depending on the data received, regulates the suction unit 30 so as to keep the oxygen concentration constant inside the mixing chamber 5.

According to a preferred embodiment the volumetric percentage of the oxygen inside the mixing chamber 5 is substantially maintained at the constant value of 18.5.

The control device 4 further comprises a timer 32 and a data processing unit 33, electrically connected to each other. The data processing unit 33, multiplying the flow of the gaseous substances leaving the mixing chamber 5 by the difference between the oxygen concentration inside the mixing chamber 5 and the oxygen concentration in the air coming from the surrounding environment, finds the oxygen consumption. The data processing unit 33 can find both the instantaneous oxygen consumption and the mean consumption in a predetermined time interval.

The data processing unit 33, using the algorithm

$$C.O. = 7\,VO_2 + 5\,,$$

in which C.O. is the cardiac output and $VO_2$ is the consumed oxygen value (A.C. Barger, V. Richards, J. Metcalfe and B. Günter, Regulation of circulation during exercise, Am. J. Physiol. 184: 613-623, 1956) (A.V. Bock, C. Vancaulaert, D.B. Dill, A. Fölling and L.M. Hurxthal, Dynamical changes occurring in man at work, J. Physiol. 66: 136, 1928), also finds the cardiac output.

The control device 4 further comprises a storage unit 34 which collects the data received from the control device 4 and the data received from the processing unit 33 and subdivides them according to subject 2 so as to create a chart for each subject 2. Each chart gives the data of all the tests carried out by a respective subject 2 so that the changes in behaviour of the subject 2 in different conditions and at different times may be observed.

The machine 1 further comprises a screen 35 electrically connected to the control device 4 on which are displayed the data received from the control device 4 and the data obtained by the processing unit 33. These data may relate to the test that the subject 2 is carrying out and/or they may be contained in one of the charts.

The machine 1 further comprises a printer 36 electrically connected to the control device 4 and with which the data received from the control device 4 and the data obtained by the processing unit 33 may be printed on sheets. These data may relate to the test that the subject 2 is carrying out and/or they may be contained in one of the charts.

According to a possible embodiment the machine 1 comprises means 37, of a prior art and schematically illustrated, electrically connected to the control device 4, for measuring the power dissipated by the subject 2 during measurement. In particular, if it is decided to take the measurements while the subject 2 is pedalling on a fixed cycle (cyclette®), said means 37 will be power meters suited for measuring the power expressed by the pedal strokes of the subject 2.

According to a further embodiment, the unit 33 processes graphs which relate the oxygen consumption and/or the cardiac output with the time and/or the effort made by the subject 2. These graphs may be displayed on the screen 35 and/or printed on sheets with the printer 36.

During the procedure, the subject 2 is positioned with his mouth and nose in front of the base 20 of the element 15 so that the space between the face of the subject 2 and the base 20 itself is such as to allow the exhaled air and the air coming from the outside environment to enter the inside of the mixing chamber 5.

When the subject 2 begins the test the suction unit 30 is already active at a minimum speed.

The exhaled air and the air coming from the outside environment are sucked into the entrance conveyor 14 and pass through the pipe 17 into the mixing chamber 5. The oxygen percentage inside the mixing chamber 5 is measured by the concentration detector 12. Depending on the signals received from the concentration detector 12, the control device 4 commands the suction unit 30 in order to regulate the flow of the gaseous substances in inverse proportion to the oxygen concentration inside the mixing chamber 5. In practice, the more the volumetric percentage of the oxygen inside the mixing chamber 5 tends to decrease with respect to the value 18.5%, the greater the flow of the gaseous substances will be.

The gaseous substances leaving the chamber 5 pass, in succession, from the section 27, the section 28 and the section 29 through the flow meter 31 and are finally released in the outside environment.

The measurement of the flow of gaseous substances taken by the flow meter 31 is sent to the control device 4.

The unit 33 multiplies the flow of the gaseous substances leaving the chamber 5 by the difference between the oxygen concentration inside the mixing chamber 5 and the oxygen concentration in the air coming from the surrounding environment, finding both the instantaneous oxygen consumption and the mean consumption with respect to a predetermined time interval.

The data processing unit 33, using the algorithm

$$C.O. = 7\ VO_2 + 5,$$

also finds the cardiac output, which may be calculated at a particular time or monitored with respect to a predetermined time interval.

The data received from the control device 4 and the data obtained by the processing unit 33 may be displayed on the screen 35 and/or memorised by the storage unit 34 so as to create a chart for each subject 2.

According to a possible embodiment the chamber 5 comprises inside it some cross members 38, illustrated with dotted lines in figure 1, defined at the hole 7, which act as flow deviators to disperse the gaseous substances homogeneously inside the mixing chamber 5 itself.

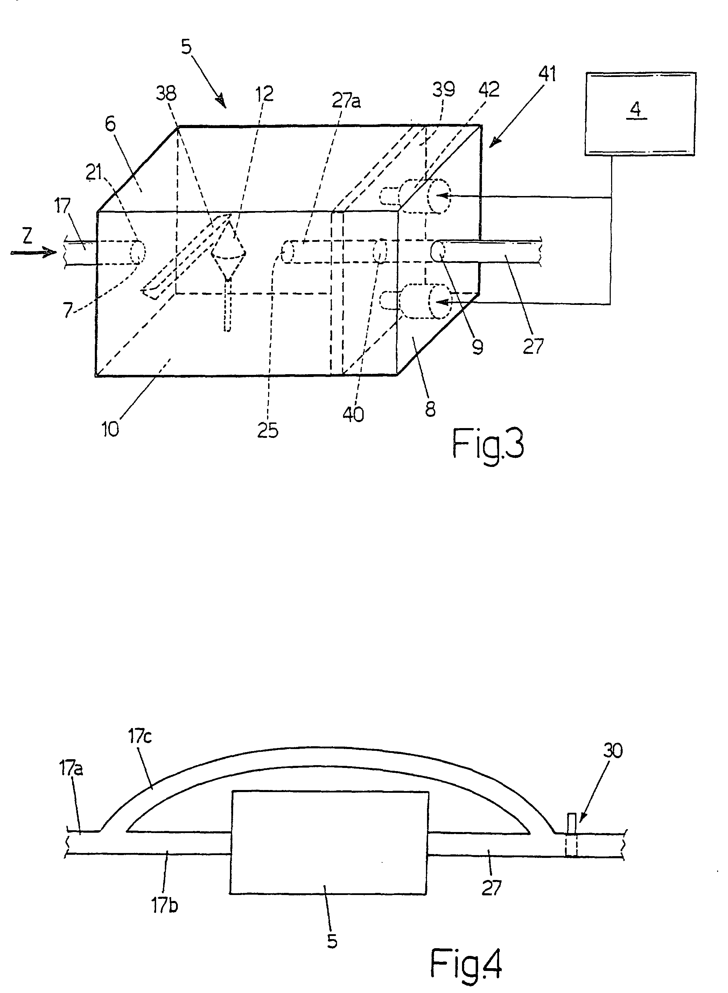

A variation of a detail of the chamber 5 is illustrated in figure 3. According to the variation the chamber 5 is equipped with a wall 39 inside the chamber 5 itself. The wall 39 is parallel to and has substantially the same shape and dimensions as the wall 8. The wall 39 has a hole 40 coaxial with the holes 7 and 9, it is mobile along the direction of the axis Z and is moved by a movement device 41, commanded by the control device 4. The movement device 41 comprises two pairs of fluidodynamic cylinders 42. The first end 25 of the pipe 24 is defined inside the chamber 5. A portion 27a of the section 27 is defined inside the chamber 5 and passes through the hole 40 in the mobile wall 39. The control device 4 commands the movement device 41 so that the mixing volume of the gaseous substances coming from the conveyor 14 is variable. In particular the volume of the chamber 5 is increased in proportion to the volume of the gaseous substances exhaled by the subject 2 during the procedure with increasing effort. In this way, even when the subject 2, at the peak of his effort, exhales large volumes of gaseous substances, the signal which the concentration detector 12 sends to the control device 4 remains more stable and is not liable to great oscillations.

According to a possible variation the detector 12 is placed on the outside of the chamber 5 to which it is connected by means of a pipe 43, illustrated in figure 1 with dotted lines. Through the pipe 43 a sample of the gaseous substances is taken from the chamber 5 and transported to the detector 12. A pump (not illustrated), defined at the detector 12, sucks up the sample.

According to an embodiment not illustrated the detector 12 comprises two sensors suited for measuring respectively the oxygen concentration and the carbon dioxide concentration. In this case the data processing unit 33 also calculates the ratio R between carbon dioxide production and oxygen consumption.

Figure 4 schematically illustrates a further embodiment according to which the pipe 17 comprises three sections 17a, 17b, 17c, connected to one another so as to form a substantially Y-shaped pipe. In particular the section 17a comprises the end 18 and it forks to form the sections 17b and 17c. The section 17b comprises the end 21 and is inserted through the hole 7 into the mixing chamber 5. The section 17c is connected directly to the outlet conveyor 23 at the section 27 downstream from the hole 8 and upstream from the suction unit 30. In this way only one portion of the gaseous substances sucked up through the conveyor 14 passes through the chamber 5 which may therefore be of small dimensions.

From the above explanation it is clear that this invention may be advantageously applied in the field of human physiology, offering the possibility of solving various problems. It is in fact possible to measure oxygen consumption and cardiac output using precise oxygen analysers which, however, have relatively long response times and therefore avoid the use of excessively complex oxygen analysers (for example mass spectrometers) which also have the inconvenience of requiring sophisticated calibration by skilled personnel.

The procedure described eliminates the need to perfectly retime the ventilation and flow signals, reducing the consequent error.

This invention further enables the above-mentioned measurements to be taken without the use of masks and mouthpieces for collecting the air exhaled by the subject 2. In this way it is no longer necessary to carry out the complex and expensive procedures of sterilising the accessories or using disposable material, which is not always adequate and has a high cost. Finally, thanks to this invention, phenomena of irregular breathing are avoided, which are due to the fact that some patients do not support the mask or mouthpiece well, so that sometimes the measurements are significantly distorted. In particular, this invention is ideal for subjects who are weak and in "basal" conditions of rest, but it guarantees performances suitable for many diagnostic requirements during procedures of programmed effort on patients with heart disease and on athletes.

**Claims**

1. Machine (1) for measuring the oxygen consumption of a living creature (2) comprising at least one mixing chamber (5) with at least one entrance zone (7) and at least one outlet zone (9), said machine (1) being **characterised in that** it comprises suction means (30) for generating a flow of gaseous substances through said entrance zone (7), from the outside of said mixing chamber (5) to the inside of the mixing chamber, and through said outlet zone (9), from the inside of said mixing chamber (5) to the outside of said mixing chamber (5), detecting means (12) for measuring the concentration of at least one first gaseous substance inside the mixing chamber (5), at least one measuring means (31) for measuring the flow of gaseous substances, a control system (4) for commanding the suction means (30) so as to keep substantially constant the concentration of said first gaseous substance inside said mixing chamber (5), data processing means (33) for finding the oxygen consumption by the living creature (2) as a function of said flow of gaseous substances; the flow of gaseous substances comprising the substances exhaled by the living creature (2) and the air coming from the outside environment.

2. Machine (1) according to claim 1, wherein said measuring means (31) comprises at least one flow detector (31) for measuring the flow of gaseous substances from the inside of said mixing chamber (5) to the outside of said mixing chamber (5).

3. Machine according to claim 2, wherein the data processing means (33) is designed to multiply the flow of substances leaving the mixing chamber (5) by the difference between the oxygen concentration inside the mixing chamber (5) and the oxygen concentration in the air coming from the outside environment so as to find the oxygen consumption by the living creature (2).

4. Machine (1) according to any one of the previous claims, wherein said mixing chamber (5) has a variable volume.

5. Machine (1) according to any one of the previous claims, wherein said first gaseous substance is oxygen.

6. Machine (1) according to claims 1 or 2, wherein said detecting means (12) are designed for measuring a concentration chosen in the group consisting in: the oxygen concentration, the carbon dioxide concentration and a combination of these.

7. Machine (1) according to any one of the previous claims, and comprising at least one conveyor means (14) for conveying the gaseous substances from the outside of said mixing chamber (5) to the inside of said mixing chamber (5); said conveyor means (14) comprising a funnel-shaped section (15).

8. Machine (1) according to claim 7, wherein the largest internal diameter of the funnel-shaped section (15) is larger than the internal diameter of the mouth, or than the diameter of the jet (E) of gaseous substances exhaled by the living creature (2), so as to define a ring-shaped crown through which the air (F) coming from the outside environment is sucked in; the surface of the base with a larger internal diameter than the funnel-shaped section (15) being at a predetermined distance from the mouth.

9. Machine (1) according to any one of the previous claims, and comprising means for detecting (37) the powers dissipated by the living creature (2).

10. Machine (1) according to one of the previous claims, and comprising a timer (32).

11. Machine (1) according to any one of the previous claims, and comprising means for finding (33) the cardiac output of the living creature (2) as a function of the oxygen consumption.

12. Machine (1) according to any one of the previous claims, comprising means for the display (35) of data chosen in the group consisting in: mean oxygen consumption, instantaneous oxygen consumption, mean production of carbon dioxide, instantaneous production of carbon dioxide, ratio (R) between oxygen consumption and carbon dioxide production, mean cardiac output, instantaneous cardiac output, mean flow of gaseous substances, instantaneous flow of gaseous substances, instantaneous dissipated power, mean dissipated power and a combination of these.

13. Machine (1) according to any one of the previous claims, comprising means for the storage (34) of data chosen in the group consisting in: the mean oxygen consumption, instantaneous oxygen consumption, mean production of carbon dioxide, instantaneous production of carbon dioxide, ratio (R) between oxygen consumption and carbon dioxide production, mean cardiac output, instantaneous cardiac output, mean flow of gaseous substances, instantaneous flow of gaseous substances, instantaneous dissipated power, mean dissipated power and a combination of these.

14. Machine (1) according to any one of the previous claims, and comprising means for printing (36) data chosen in the group consisting in: mean oxygen consumption, instantaneous oxygen consumption, mean production of carbon dioxide, instantaneous production of carbon dioxide, ratio (R) between oxygen consumption and carbon dioxide production, mean cardiac output, instantaneous cardiac output, mean flow of gaseous substances, instantaneous flow of gaseous substances, instantaneous dissipated power, mean dissipated power and a combination of these.

15. Method for measuring oxygen consumption which provides suction of the substances exhaled by a living creature (2) into a machine (1) for measurement equipped with a mixing chamber (5); said method being **characterised in that** it provides the suction, together with the substances exhaled by the living creature (2), of the air coming from the outside environment; measurement of the concentration of at least a first gaseous substance inside the mixing chamber (5); variation of the flow of substances entering and/or leaving the measuring machine (1) in such a way as to maintain substantially constant the concentration of the at least first gaseous substance inside the mixing chamber (5); measurement of the flow of substances entering and/or leaving the measuring machine (1); calculation of the oxygen consumption as a function of the flow of substances entering and/or leaving the measuring machine (1).

16. Method according to claim 15, wherein the oxygen consumption by the living creature (2) is obtained by multiplying the flow of substances leaving the measuring machine (1) by the difference between the oxygen concentration inside the mixing chamber (5) and the oxygen concentration in the air coming from the environment surrounding the mixing chamber (5).

17. Method according to claim 15 or 16, wherein the first gaseous substance of which the concentration is measured and of which the concentration inside the mixing chamber (5) is kept substantially constant, is oxygen.

18. Method according to any one of the claims from 15 to 17, and which provides measurement of the power dissipated by the living creature (2).

19. Method according to any one of the claims from 15 to 18, and which provides calculation of the cardiac output of the living creature (2) as a function of the oxygen consumption of the living creature (2).

20. Method according to claim 19, wherein, depending on the oxygen consumption ($VO_2$) and on the algorithm:

$$C.O. = 7\, VO_2 + 5\,,$$

the cardiac output (C.O.) of the living creature (2) is obtained.

21. Method according to any one of the claims from 15 to 20, and which provides the display of any one of: mean oxygen consumption, instantaneous oxygen consumption, mean production of carbon dioxide, instantaneous production of carbon dioxide, mean cardiac output, instantaneous cardiac output, mean flow of gaseous substances, instantaneous flow of gaseous substances, instantaneous dissipated power, mean dissipated power or a combination of these.

22. Method according to any one of the claims from 15 to 21, and which provides the storage of any one of: mean oxygen consumption, instantaneous oxygen, consumption, mean production of carbon dioxide, instantaneous production of carbon dioxide, mean cardiac output, instantaneous cardiac output, mean flow of gaseous substances, instantaneous flow of gaseous substances, instantaneous dissipated power, mean dissipated power or a combination of these.

23. Method according to any one of the claims from 15 to 22, and which provides the printing of any one of: mean oxygen consumption, instantaneous oxygen consumption, mean production of carbon dioxide, instantaneous production of carbon dioxide, mean cardiac output, instantaneous cardiac output, mean flow of gaseous substances, instantaneous flow of gaseous substances, instantaneous dissipated power, mean dissipated power or a combination of these.

24. Method according to claim 22 or 23, and which provides the storage of the oxygen consumption, the cardiac output, the flow of gaseous substances, the power dissipated by the living creature in a single chart so that, by analysing the chart, it is possible to assess the variations of each parameter.

**Patentansprüche**

1. Apparat (1) zur Messung des Sauerstoffverbrauchs eines Lebewesens (2), welcher wenigstens eine Mischkammer (5) mit wenigstens einem Eingangsbereich (7) und wenigstens einem Auslassbereich (9) umfasst, wobei der besagte Apparat dadurch charakterisiert ist, dass er Folgendes umfasst: Saugvorrichtungen (30), um damit einen Strom von gasförmigen Substanzen durch den besagten Eingangsbereich (7), von der Außenseite der besagten Mischkammer (5) in das Innere der Mischkammer, und durch den besagten Auslassbereich (9), aus dem Inneren der besagten Mischkammer (5) an die Außenseite der besagten Mischkammer (5), zu erzeugen, Ermittlungsvorrichtungen (12), um damit die Konzentration wenigstens einer ersten, gasförmigen Substanz im Inneren der Mischkammer (5) zu messen, wenigstens ein Messgerät (31), um damit den Strom der gasförmigen Substanzen zu messen, ein Regelsystem (4), um damit die Saugvorrichtungen (30) zu steuern, um so die Konzentration der besagten ersten, gasförmigen Substanz im Inneren der besagten Mischkammer (5) im Wesentlichen konstant zu halten, Vorrichtungen zu Datenverarbeitung (33), um damit den Sauerstoffverbrauch des Lebewesens (2) als eine Funktion des besagten Stroms der gasförmigen Substanzen zu ermitteln; wobei der Strom der gasförmigen Substanzen diejenigen Substanzen, die von dem Lebewesen (2) ausgeatmet werden, sowie die Luft, die aus der äußeren Umgebung stammt, umfasst.

2. Apparat (1) gemäß Anspruch 1, bei welchem das besagte Messgerät (31) wenigstens eine Strömungs-Ermittlungsvorrichtung (31) umfasst, um damit den Strom der gasförmigen Substanzen, aus dem Inneren der besagten Mischkammer (5) an die Außenseite der besagten Mischkammer (5), zu messen.

3. Apparat gemäß Anspruch 2, bei welchem die Vorrichtung zur Datenverarbeitung (33) so gestaltet ist, dass sie den Strom der gasförmigen Substanzen, welcher die Mischkammer (5) verlässt, mit der Differenz zwischen der Sauerstoffkonzentration Innerhalb der Mischkammer (5) und der Sauerstoffkonzentration in der Luft, die aus der äußeren Umgebung stammt, multipliziert, um so den Sauerstoffverbrauch des Lebewesens (2) zu ermitteln.

4. Apparat (1) gemäß irgendeinem der vorherigen Ansprüche, bei welchem die besagte Mischkammer (5) ein variables Volumen hat.

5. Apparat (1) gemäß irgendeinem der vorherigen Ansprüche, bei welchem die besagte erste, gasförmige Substanz Sauerstoff ist.

6. Apparat (1) gemäß den Ansprüchen 1 oder 2, bei welchem die besagten Ermittlungsvorrichtungen (12) so gestaltet sind, dass sie eine Konzentration messen, die aus der Gruppe, welche aus der Sauerstoffkonzentration, der Kohlendioxydkonzentration und einer Kombination daraus besteht, ausgesucht wurde.

7. Apparat (1) gemäß irgendeinem der vorherigen Ansprüche, welcher wenigstens ein Beförderungsmittel (14) umfassen, um damit die gasförmigen Substanzen von der Außenseite der besagten Mischkammer (5) in das Innere der besagten Mischkammer (5) zu transportieren: wobei die besagten Beförderungsmittel (14) einen trichterförmigen Abschnitt (15) umfassen.

8. Apparat (1) gemäß Anspruch 7, bei welchem der größte Innendurchmesser des trichterförmigen Abschnitts (15) größer als der Innendurchmesser des Mundes ist, oder als der Durchmesser des Ausstroms (E) der gasförmigen Substanzen, die von dem Lebewesen (2) ausgeatmet werden, um so einen ringförmigen Scheitelrand festzulegen, durch welchen die Luft (F), die aus der äußeren Umgebung stammt, eingesaugt wird; wobei sich die Oberfläche der Fassung, mit einem größeren Innendurchmesser als ihn der tunnelförmige Abschnitt (15) hat, in einer festgelegten Distanz zum Mund befindet.

9. Apparat (1) gemäß irgendeinem der vorherigen Ansprüche, welcher Vorrichtungen zur Ermittlung (37) der Energie, die von dem Lebewesen (2) verbraucht wird, umfasst.

10. Apparat (1) gemäß irgendeinem der vorherigen Ansprüche, welcher einen Timer (32) umfasst.

11. Apparat (1) gemäß irgendeinem der vorherigen Ansprüche, welcher Vorrichtungen zum Ermitteln (33) der Herzleistung des Lebewesens (2) als eine Funktion des Sauerstoffverbrauchs umfasst.

12. Apparat (1) gemäß irgendeinem der vorherigen Ansprüche, welcher Vorrichtungen zur Wiedergabe (35) von Daten umfasst, die aus der Gruppe ausgewählt wurden, welche aus Folgendem besteht: mittlerer Sauerstoffverbrauch, augenblicklicher Sauerstoffverbrauch, mittlere Kohlendioxydproduktion, augenblickliche Kohlendioxydproduktion, Verhältnis (R) zwischen Sauerstoffverbrauch und Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch und eine Kombination daraus.

**13.** Apparat (1) gemäß irgendeinem der vorherigen Ansprüche, welcher Vorrichtungen für die Speicherung (34) von Daten umfasst, die aus der Gruppe ausgewählt wurden, welche aus Folgenden besteht: der mittlere Sauerstoffverbrauch, augenblicklicher Sauerstoffverbrauch, mittlere Kohlendioxydproduktion, augenblickliche Kohlendioxydproduktion, Verhältnis (R) zwischen Sauerstoffverbrauch und Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch und eine Kombination daraus.

**14.** Apparat (1) gemäß irgendeinem der vorherigen Ansprüche, welcher Vorrichtungen zum Ausdrucken (36) der Daten umfasst, die aus der Gruppe ausgewählt wurden, welche aus Folgenden besteht: mittlerer Sauerstoffverbrauch, augenblicklicher Sauerstoffverbrauch, mittlere Kohlendioxydproduktion, augenblickliche Kohlendioxydproduktion, Verhältnis (R) zwischen Sauerstoffverbrauch und Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch und eine Kombination daraus.

**15.** Verfahren zur Messung des Sauerstoffverbrauchs, welches das Ansaugen der Substanzen, die von einem Lebewesen (2) in einen Apparat (1) für Messungen, der mit einer Mischkammer (5) ausgestattet ist, hinein ausgeatmet werden, bereit stellt; das besagte Verfahren ist dadurch charakterisiert, dass es Folgendes bereit stellt: das Ansaugen der Luft, die aus der äußeren Umgebung stammt, zusammen mit den Substanzen, die von dem Lebewesen (2) ausgeatmet werden; das Messen der Konzentration von wenigstens einer ersten, gasförmigen Substanz innerhalb der Mischkammer (5); die Veränderung des Stroms von Substanzen, der in die Messapparatur (1) eintritt und/oder sie verlässt, auf eine solche Art und Weise, dass die Konzentration der wenigstens einen ersten, gasförmigen Substanz innerhalb der Mischkammer (5) im Wesentlichen konstant gehalten wird; das Messen des Stroms von Substanzen, der in die Messapparatur (1) eintritt und/oder sie verlässt; die Berechnung des Sauerstoffverbrauchs als eine Funktion des Stroms von Substanzen, der in die Messapparatur (1) eintritt und/oder sie verlässt.

**16.** Verfahren gemäß Anspruch 15, in welchem der Sauerstoffverbrauch des Lebewesens (2) durch die Multiplikation des Stroms von Substanzen, der die Messapparatur (1) verlässt, mit der Differenz zwischen der Sauerstoffkonzentration im Inneren der Mischkammer (5) und der Sauerstoffkonzentration in der Luft, die aus der Umgebung, welche die Mischkammer (5) umgibt, stammt, erhalten wird.

**17.** Verfahren gemäß Anspruch 15 oder 16, in welchem die erste, gasförmige Substanz, deren Konzentration gemessen wird, und deren Konzentration im Inneren der Mischkammer (5) im Wesentlichen konstant gehalten wird, Sauerstoff ist.

**18.** Verfahren gemäß irgendeinem der Ansprüche von 15 bis 17, welches das Messen der Energie, die das Lebewesen (2) verbraucht, bereit stellt.

**19.** Verfahren gemäß irgendeinem der Ansprüche von 15 bis 18, welches die Berechnung der Herzleistung des Lebewesens (2) als eine Funktion des Sauerstoffverbrauchs des Lebewesens (2) bereit stellt.

**20.** Verfahren gemäß Anspruch 19, in welchem, abhängig vom Sauerstoffverbrauch ($VO_2$) und der Gleichung:

$$C.O = 7\ VO_2 + 5\ .$$

die Herzleistung (C.O.) des Lebewesens (2) erhalten wird.

**21.** Verfahren gemäß irgendeinem der Ansprüche von 15 bis 20, welches die Wiedergabe von irgendeinem der folgenden Werte bereit stellt: mittlerer Sauerstoffverbrauch, augenblicklicher Sauerstoffverbrauch, mittlere Kohlendioxydproduktion, augenblickliche Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch oder eine Kombination daraus.

**22.** Verfahren gemäß irgendeinem der Ansprüche von 15 bis 21, welches die Speicherung von irgendeinem der folgenden Werte bereit stellt; mittlerer Sauerstoffverbrauch, augenblicklicher Sauerstoffverbrauch, mittlere Kohlendioxydproduktion. augenblickliche Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch oder eine Kombination daraus.

**23.** Verfahren gemäß Irgendeinem der Ansprüche von 15 bis 22, welches das Ausdrucken von irgendeinem der folgenden Werte bereit stellt: mittlerer Sauerstoffverbrauch, augenblicklicher Sauerstoffverbrauch, mittlere Kohlendioxydproduktion, augenblickliche Kohlendioxydproduktion, mittlere Herzleistung, augenblickliche Herzleistung, mittlerer Strom von gasförmigen Substanzen, augenblicklicher Strom von gasförmigen Substanzen, augenblicklicher Energieverbrauch, mittlerer Energieverbrauch oder eine Kombination daraus.

24. Verfahren gemäß Anspruch 22 oder 23, welches die Speicherung des Sauerstoffverbrauchs, der Herzleistung, des Stroms von gasförmigen Substanzen und des Energieverbrauchs des Lebewesens in einem einzigen Kurvenblatt bereit stellt, so dass es durch die Analyse dieses Kurvenblattes möglich ist, die Veränderungen eines jeden Parameters abzuschätzen.

**Revendications**

1. Machine (1) pour mesurer la consommation d'oxygène d'une créature vivante (2) comprenant au moins une chambre de mélange (5) comportant au moins une zone d'entrée (7) et au moins une zone de sortie (9), ladite machine (1) étant **caractérisée en ce qu'**elle comprend des moyens d'aspiration (30) pour produire un écoulement de substances gazeuses dans ladite zone d'entrée (7), depuis l'extérieur de ladite chambre de mélange (5) vers l'intérieur de la chambre de mélange, et dans ladite zone de sortie (9), depuis l'intérieur de ladite chambre de mélange (5) vers l'extérieur de ladite chambre de mélange (5), des moyens de détection (12) pour mesurer la concentration d'au moins une première substance gazeuse à l'intérieur de la chambre de mélange (5), au moins un moyen de mesure (31) pour mesurer l'écoulement de substances gazeuses, et un système de commande (4) pour commander les moyens d'aspiration (30) de manière à maintenir essentiellement constante la concentration de ladite première substance gazeuse à l'intérieur de ladite chambre de mélange (5), des moyens de traitement (33) pour déterminer la consommation d'oxygène par la créature vivante (2) en fonction dudit écoulement de substances gazeuses; l'écoulement de substances gazeuses comprenant les substances exhalées par la créature vivante (2) et l'air arrivant de l'environnement extérieur.

2. Machine (1) selon la revendication 1, dans laquelle lesdits moyens de mesure (31) comprennent au moins un détecteur d'écoulement (31) pour mesurer l'écoulement de substances gazeuses depuis l'intérieur de ladite chambre de mélange (5) vers l'extérieur de ladite chambre de mélange (5).

3. Machine selon la revendication 2, dans laquelle les moyens de traitement de données (33) sont conçus de manière à multiplier l'écoulement de substances quittant la chambre de mélange (5) par la différence entre la concentration en oxygéne à l'intérieur de la chambre de mélange (5) et la concentration en oxygène dans l'air arrivant de l'environnement extérieur de manière à trouver la consommation d'oxygène par la créature vivante (2).

4. Machine (1) selon l'une quelconque des revendications précédentes, dans laquelle ladite chambre de mélange (5) possède un volume variable.

5. Machine (1) selon l'une quelconque des revendications précédentes, dans laquelle ladite première substance gazeuse est de l'oxygène.

6. Machine (1) selon la revendication 1 ou 2, dans laquelle lesdits moyens de détection (12) sont conçus de manière à mesurer une concentration choisie dans le groupe comprenant: la concentration en oxygène, la concentration en gaz carbonique et une combinaison de ces concentrations.

7. Machine (1) selon l'une quelconque des revendications précédentes, comprenant au moins un moyen convoyeur (14) pour convoyer les substances gazeuses de l'extérieur de ladite chambre de mélange (5) à l'intérieur de ladite chambre de mélange (5); ledit moyen convoyeur (14) comprenant une section en forme d'entonnoir (15).

8. Machine (1) selon la revendication 7, dans laquelle le diamètre intérieur maximum de la section en forme d'entonnoir (15) est supérieur au diamètre intérieur de la bouche ou au diamètre du jet (E) de substances gazeuses exhalées par la créature vivante (2) de manière à définir une couronne de forme annulaire, à l'intérieur de laquelle est aspirée l'air (F) arrivant de l'environnement extérieur; la surface de la base possédant un diamètre intérieur supérieur à celui de la section en forme d'entonnoir (15) étant située à une distance prédéterminée de la bouche.

9. Machine (1) selon l'une quelconque des revendications précédentes, et comprenant des moyens pour détecter (37) la puissance dissipée par la créature vivante (2).

10. Machine (1) selon l'une quelconque des revendications précédentes, et comprenant une minuterie (32).

11. Machine (1) selon l'une quelconque des revendications précédentes, et comprenant des moyens (33) pour déterminer le débit cardiaque de la créature vivante (2) en fonction de la consommation d'oxygène.

**12.** Machine (1) selon l'une quelconque des revendications précédentes, comprenant des moyens (35) pour l'affichage de données, choisies dans le groupe comprenant: la consommation moyenne d'oxygène, la consommation instantanée d'oxygène, la production moyenne de gaz carbonique, la production instantanée de gaz carbonique, le rapport (R), entre la consommation d'oxygène et la production de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

**13.** Machine (1) selon l'une quelconque des revendications précédentes, comprenant des moyens (34) pour mémoriser des données choisies dans le groupe comprenant: la consommation moyenne d'oxygène, la consommation instantanée d'oxygène, la production moyenne de gaz carbonique, la production instantanée de gaz carbonique, le rapport (R) entre la consommation d'oxygène et la production de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

**14.** Machine (1) selon l'une quelconque des revendications précédentes, et comprenant des moyens (36) pour imprimer des données choisies dans le groupe comprenant: la consommation moyenne d'oxygène, la consommation instantanée d'oxygène, la production moyenne de gaz carbonique, la production instantanée de gaz carbonique, le rapport (R) entre la consommation d'oxygène et la production de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

**15.** Procédé pour mesurer la consommation d'oxygène, qui réalise l'aspiration des substances exhalées par un être vivant (2) dans une machine de mesure (1) équipée d'une chambre de mélange (5); ledit procédé étant **caractérisé en ce qu'**il réalise l'aspiration, conjointement avec les substances exhalées par la créature vivante (2), de l'air arrivant de l'environnement extérieur; la mesure de la concentration d'au moins une première substance gazeuse à l'intérieur de la chambre de mélange (5); la variation de l'écoulement de substances pénétrant dans et/ou quittant la machine de mesure (1) de manière à maintenir essentiellement constante la concentration au moins de la première substance gazeuse à l'intérieur de la chambre de mélange (5); la mesure du débit de substances pénétrant dans et/ou quittant la machine de mesure (1); le calcul de la consommation d'oxygène en fonction du débit de substances pénétrant dans et/ou quittant la machine de mesure (1).

**16.** Procédé selon la revendication 15, selon lequel la concentration d'oxygène pour la créature vivante (2) est obtenue par multiplication de l'écoulement de substances quittant la machine de mesure (1) par la différence entre la concentration d'oxygène à l'intérieur de la chambre de mesure (5) et la concentration d'oxygène dans l'air arrivant de l'environnement entourant la chambre de mélange (5).

**17.** Procédé selon la revendication 15 ou 16, dans lequel la première substance gazeuse, dont la concentration est mesurée, et dont la concentration à l'intérieur de la chambre de mélange (5) est maintenue essentiellement constante, est l'oxygène.

**18.** Procédé selon l'une quelconque des revendications 15 à 17, et qui fournit une mesure de la puissance dissipée par la créature vivante (2).

**19.** Procédé selon l'une quelconque des revendications 15 à 18, et qui fournit un calcul du débit cardiaque de la créature vivante (2) en fonction de la consommation d'oxygène de la créature vivante (2).

**20.** Procédé selon la revendication 19, selon lequel on obtient, en fonction de la consommation d'oxygène ($VO_2$) et de l'algorithme:

$$C.O. = 7\ VO_2 + 5$$

le débit cardiaque (C.O.) de la créature vivante (2).

**21.** Procédé selon l'une quelconque des revendications 15 à 20, et qui fournit l'affichage de l'un quelconque de: la consommation moyenne d'oxygène, la consommation instantanée d'oxygène, la production moyenne de gaz carbonique, la production instantanée de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

**22.** Procédé selon l'une quelconque des revendications 15 à 21, et qui réalise la mémorisation de l'un quelconque de: la consommation moyenne d'oxygène, la consommation instantanée d'oxygène, la production moyenne de gaz carbonique, la production instantanée de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

23. Procédé selon l'une quelconque des revendications 15 à 22, et qui réalise l'impression de l'un quelconque de: la consommation moyenne d'oxygène, la consommation instantanée d'oxygène, la production moyenne de gaz carbonique, la production instantanée de gaz carbonique, le débit cardiaque moyen, le débit cardiaque instantané, le débit moyen de substances gazeuses, le débit instantané de substances gazeuses, la puissance instantanée dissipée, la puissance moyenne dissipée et une combinaison de ces éléments.

24. Procédé selon la revendication 22 ou 23, et qui réalise la mémorisation de la consommation en oxygène, du signal de sortie cardiaque, du débit de substances gazeuses, de la puissance dissipée par la créature vivante dans un seul diagramme de telle sorte que, moyennant l'analyse du diagramme, il est possible d'accéder aux variations de chaque paramètre.

Fig.1

EP 1 234 541 B1

Fig.2

Fig.3

Fig.4